Europäisches Patentamt

European Patent Office

Office européen des brevets

(19)

(11) Publication number: **0 248 639 B1**

(12) **EUROPEAN PATENT SPECIFICATION**

(45) Date of publication of patent specification: **29.09.93**  (51) Int. Cl.⁵: **A61B 17/04**, A61B 17/06, A61L 17/00

(21) Application number: **87304876.3**

(22) Date of filing: **02.06.87**

(54) **A method of storing a suture and a package containing the suture.**

(30) Priority: **02.06.86 JP 125909/86**

(43) Date of publication of application:
**09.12.87 Bulletin 87/50**

(45) Publication of the grant of the patent:
**29.09.93 Bulletin 93/39**

(84) Designated Contracting States:
**CH DE FR GB IT LI NL**

(56) References cited:
**BE-A- 872 208**
**GB-A- 2 008 135**
**US-A- 4 135 622**
**US-A- 4 300 565**

(73) Proprietor: **MITSUI TOATSU CHEMICALS, Inc.**
**2-5 Kasumigaseki 3-chome**
**Chiyoda-Ku Tokyo 100(JP)**

(72) Inventor: **Takayanagi, Hiroshi**
**300, Hirabaru**
**Omuta-shi Fukuoka-ken(JP)**
Inventor: **Kobayashi, Tadashi**
**101, Shozanmachi**
**Omuta-shi Fukuoka-ken(JP)**

(74) Representative: **Harvey, David Gareth et al**
**Graham Watt & Co. Riverhead**
**Sevenoaks Kent TN13 2BN (GB)**

EP 0 248 639 B1

# EP 0 248 639 B1

## Description

This invention relates to a method for the storage of a surgical suture obtained from a polyglycolic acid or a glycolic acid copolymer containing ℓ-lactic acid in an amount not greater than 15 mole % based on glycolic acid, and also to a package containing the suture.

Polyglycolic acid, polylactide and copolymers of glycolic acid or lactide are solid, bioabsorbable and hydrolyzable polymers and are used for surgical purposes such as sutures. As a polymer for such sutures, polyglycolic acid is usually obtained, for example by polymerizing at a high temperature of 200°C or higher glycolic acid in the presence of a polymerization catalyst such as stannous chloride or stannous stearate in an amount of about 0.0005 - 0.0025 wt.% based on the monomer and a small amount of a saturated aliphatic alcohol, e.g., lauryl alcohol as a cocatalyst as disclosed in Japanese Patent Publication No. 13595/1970.

On the other hand, a copolymer of glycolide and ℓ-lactide is obtained, for example, by polymerizing at a high temperature of 200°C or higher a mixture of 85 - 90 mole % of glycolide and 10 - 15 mole % of ℓ-lactide in the presence of stannous octoate as a single polymerization catalyst in an amount of 1 mole per 50,000 - 150,000 moles of the monomers, namely, in an amount of 0.008 - 0.0027 wt.% of the monomers as disclosed in Japanese Patent Publication No. 14688/1981.

Such polyglycolic acid and copolymers of glycolic acid have been known to have many advantages as surgical sutures over natural catguts. Some of these advantages however turn to disadvantages in certain instances. For example, the meritorious hydrolyzability may cause problems upon storage of sutures. Especially, the tensile strength of polyglycolic acid drops rapidly and its biodegradability is impaired when it is exposed to water or moisture (hereinafter collectively referred to as "moisture"). It has hence been considered to be necessary to shut out moisture completely for the storage of sutures of polyglycolic acid.

Regarding the storage of sutures obtained from polyglycolic acid, Japanese Patent Publication No. 44754/1974 teaches the storing of a suture by using a completely moisture-impervious container such as an aluminum foil container upon packing a suture, heating and drying the suture and container under conditions free of air for a time period sufficient to reduce the water content to substantially zero, thereby to drive out moisture completely, and then vacuum-packing the suture or filling the interior of the container with an inert gas such as nitrogen or argon thus packing the suture under moisture-free conditions within the container.

Such packing requiring strict exclusion of moisture is however a very time-consuming and inconvenient method. Even when sutures are stored under controlled conditions within such perfect packages, moisture may still penetrate into the packages depending on the storage environment. Penetration of moisture, even in an extremely small amount, unavoidably degrades the sutures so that their quality varies from one suture to another.

An object of this invention is to provide a method for effectively storing a suture obtained from polyglycolic acid or a copolymer of glycolic acid, by use of a package from which moisture has been removed to a degree as practised in the ordinary storage of drugs, and without relying upon a controlled storage method requiring strict exclusion of all moisture. The invention comprehends a method for storing a suture in accordance with claim 1.

The present inventors have conducted an extensive investigation in order to achieve the above object. As a result, it has been found for the first time that a suture can be stored without relying upon a controlled storage method requiring strict exclusion of all moisture, so long as the suture has specific physical properties, no matter whether the suture is made of polyglycolic acid or a copolymer of glycolic acid.

This invention therefore provides a method for the storage of a suture, which permits use of the suture without partial or entire loss of the biodegradability of the suture even after a usual shelf period and without relying upon any controlled storage method requiring strict exclusion of moisture. The method is applicable so long as the suture is one obtained from polyglycolic acid or a copolymer of glycolic acid and its viscosity average polymerization degree expressed typically in terms of its intrinsic viscosity $[\eta]$ is not lower than a particular value.

In other words, this invention provides a method for storing a suture, which has been obtained from polyglycolic acid or a copolymer of glycolic acid, by controlling the intrinsic viscosity $[\eta]$ of the suture.

As stated above, we have found that it is possible to store a suture satisfactorily without having to strictly exclude all moisture. In contrast, US-A-4,135,622 discloses that sutures packaged in films of SARAN (Registered Trade Mark) and SCOTCH PAK (Registered Trade Mark) were inadequately protected and disintegrated after a relatively short storage time (e.g. only 42 days). US-A-4,135,622 teaches that sutures should be stored in packages made from plastics laminate films containing a metallic foil layer.

2

The present invention provides a method for storing a suture, the method comprising selection of a suture obtained by spinning a polyglycolic acid polymer or a glycolic acid copolymer of glycolide and $\ell$-lactide in an amount not greater than 15% by weight based on the glycolide, said suture having an intrinsic viscosity of at least 0.85; sterilizing and drying the suture; and hermetically packaging the suture together with dry air having a moisture content of 0.1% by weight or below in a container which had been stored in dry air in advance, the container being made of a substantially moisture-impervious material, but not a completely moisture-impervious material such as aluminum foil.

According to the method of this invention, a conventional procedure may be followed upon packing a suture. No problems arise even if a package adapted to pack the suture allows moisture to penetrate, so long as the amount of moisture is in an extremely small amount. It is sufficient for the package so long as the package does not permit substantial penetration of moisture thereinto. A suture can be stored satisfactorily in a simple package with a filling of dry air of such quality as routinely available during a packing operation.

The invention comprehends a package of a suture of a polyglycolic acid or a glycolic acid copolymer of glycolide and $\ell$-lactide, the package being produced in accordance with the above method.

According to the method of this invention for the storage of a suture of a polyglycolic acid or a glycolide-$\ell$-lactide copolymer, the suture is selected for storage on the basis of its intrinsic viscosity, so as to store it with a high polymerization degree. The packaged material therefore undergoes little degradation due to changes through passage of time during its storage. Even after the lapse of a usual storage period, a packaged suture can still be used without any problems.

Embodiments of the invention will now be explained in more detail in the following non-limitative description.

Polyglycolic acid and copolymers of glycolic acid, which are useful in the practice of this invention, can be produced by using glycolide and both glycolide and $\ell$-lactide as raw materials respectively. The copolymers of glycolic acid can each be obtained by copolymerizing glycolide and $\ell$-lactide in an amount not greater than 15 mole %. Any copolymers containing $\ell$-lactide in amounts greater than this upper limit cannot provide sutures having the desired $[\eta]$.

In the method of this invention, the intrinsic viscosity $[\eta]$ of a suture to be stored is critical. Therefore, it is also necessary to pay attention to the process of polymerizing polyglycolic acid or a copolymer of glycolic acid. According to conventional polymerization processes like the above-described prior art polymerization processes, polymers are usually obtained with varied intrinsic viscosities $[\eta]$ or with intrinsic viscosities $[\eta]$ as low as 0.8. Sutures of such viscosities are however not effective for use in the storage method according to this invention.

Upon polymerization of polyglycolic acid and glycolic acid copolymers to be used for the production of sutures useful in the practice of this invention, it is therefore always preferred to use stannous octoate as a polymerization catalyst. Use of other catalysts, for example, a catalyst such as stannous chloride cannot provide polyglycolic acid and glycolic acid copolymers having the desired high degrees of polymerization.

The polymerization catalyst may preferably be used in an amount of 0.001 - 0.005 wt.% based on glycolide in the case of polyglycolic acid or both glycolide and $\ell$-lactide in the case of a glycolic acid copolymer. In addition, lauryl alcohol is also used as a co-catalyst and polymerization regulator. It is preferable to use lauryl alcohol in a relatively large amount, for example, 60 - 120 times the amount of stannous octoate employed as a catalyst. If lauryl alcohol is used in any amounts outside this range, it is difficult to achieve the desired physical properties.

The polymerization temperature may range from 220°C to 240°C. In other words the polymerization is carried out at a relatively high temperature.

Polyglycolic acid and copolymers of glycolic acid obtained by conducting their polymerization for 2 - 6 hours under such conditions have extremely low tin contents and their molecular weights fall within a range of from about 10,000 to 100,000. It is therefore possible to obtain polyglycolic acid and glycolic acid copolymers each of which has an intrinsic viscosity of 0.85 - 1.1 and a melt index of 1.0 - 5.0 as defined and measured below.

Intrinsic viscosity $[\eta]$

Each polymer was dissolved in a mixed solvent of 10 parts by weight of phenol and 7 parts by weight of trichlorophenol. The logarithmic viscosity of the resultant solution was measured as the intrinsic viscosity of the polymer at 30 ± 0.1°C and a concentration of 0.5% by a Ubbelohde's viscometer.

Melt index [MI]

The melt index of each polymer was measured in accordance with a method similar to the ASTM method D1238-65T published by The American Society for Testing Materials. Namely, 325 g of the polymer was extruded at 230°C through a 2.1-mm orifice of a melt indexer. The MI of the polymer was indicated by the number of grams per 10 minutes.

In the method of this invention, the intrinsic viscosity of a suture to be stored must be at least 0.85. For this purpose, polyglycolic acid or a copolymer of glycolic acid which is to be used in spinning should have an intrinsic viscosity [$\eta$] of at least 0.85, preferably 0.85 to 1.1 If [$\eta$] exceeds 1.1, no suitable extrudability can be obtained upon spinning so that the extrusion temperature must be raised to 250°C or higher. Even if an intrinsic viscosity [$\eta$] as high as about 0.95 as measured after spinning should be achieved, the polyglycolic acid or glycolic acid copolymer cannot avoid thermal degradation and molecular weight reduction so that good spinning is not feasible.

If the [$\eta$] of polyglycolic acid or a glycolic acid copolymer is lower than 0.85, more end breakages occur upon stretching and the production yield is hence reduced. Further, the [$\eta$] of the resulting suture becomes 0.8 or lower.

No problems or inconvenience arise during a usual storage period provided that the [$\eta$] of the suture employed in this invention is 0.85 or higher. In view of the formability and production yield upon spinning, sutures having intrinsic viscosities [$\eta$] of 0.85 - 0.95 as measured after spinning are preferably used. In order to obtain sutures having intrinsic viscosities [$\eta$] within the above range, polyglycolic acid or a glycolic acid copolymer having an intrinsic viscosity [$\eta$] of about 0.85 may be used depending on spinning conditions. Polyglycolic acid or a glycolic acid copolymer having an intrinsic viscosity [$\eta$] in a range of 0.9 - 1.0 is generally preferred as a polymer to be subjected to spinning.

The control of these intrinsic viscosities [$\eta$] within the above-described range can be achieved, as desired, principally by adjusting the ratio of the catalyst to the co-catalyst. If [$\eta$] in the neighborhood of 1.0 - 1.1 is desired by way of example, it is necessary to use stannous octoate in an amount of 0.001 - 0.005 wt.% based on the glycolide on the glycolide and $\ell$-lactide and lauryl alcohol in an amount above 60 times in weight the stannous octoate.

In order to obtain a suture useful in the practice of this invention from the polyglycolic acid or glycolic acid copolymer obtained in the above manner, the polyglycolic acid or glycolic acid copolymer is melt-spun through a nozzle with 8 - 40 holes by using a usual melt extruder so that multifilaments are produced.

The extrusion pressure by an extrusion screw may be 10 to 200 kg/cm$^2$, (1 to 20 N/mm$^2$) whereas the extrusion temperature may be 250°C or below, preferably 235 - 245°C in the case of polyglycolic acid and 225 - 235°C in the case of a glycolic acid copolymer in order to avoid thermal degradation. After spinning, the multifilaments are continuously guided onto a hot plate maintained at 120°C, where the multifilaments are stretched by a factor of about 4 so as to obtain good multifilaments having a tensile strength of about 6.5 g/denier (58.5 g/tex) and an intrinsic viscosity [$\eta$] of at least 0.85. The multifilaments are then braided by a method known per se and after sterilization, are hermetically sealed to provide a surgical suture.

According to the method of this invention, a suture obtained in the above-described manner is packed for storage in a container made of a substantially moisture-impervious material and is hermetically sealed with dry air inside the container. After sterilizing and drying the thus-obtained suture, the suture is packed within the substantially moisture-impervious container in an atmosphere of dry air for storage.

Any container may be used as the container without any problems so long as it is made of a material known to be substantially moisture-impervious. It is not essential to use a container made of a completely moisture-impervious material such as aluminum foil, however. Exemplary containers useful in the practice of this invention include "Scotch Pack" (trade mark), a packaging made principally of a polyester-polyethylene laminate film and used as packs for catguts, and containers made of materials such as laminate films of "Saran" (trade mark), which is a vinyl chloride-vinylidene chloride copolymer, and polyethylene materials.

The dry air used when packing a suture in accordance with the present method of this invention can be satisfactorily obtained simply by causing air to pass through a column of a conventional molecular sieve as a drying agent. The air can have a dew point of 20°C and a moisture content of $10^{-3}$ kg/kg-air. Such dry air, having a moisture content of 0.1% or lower, is sold in bombs on the market and is hence readily available.

When such dry air is used, the moisture ratio is about 5 - 10 ppm relative to the suture when the suture is packed in the form of a flattened thin layer which is the usual packing form for commercial packages.

By the method described above, there is provided a package enclosing a suture, which has been obtained from polyglycolic acid or a glycolic acid copolymer and then processed for use in surgery and has the specific intrinsic viscosity, along with dry air therein. For use in surgery, the suture is extracted from the package ready for use.

The suture is maintained stably within the package. Even after keeping through a normal shelf storage period, no substantial deterioration is observed with respect to performance such as biodegradability.

The present invention will hereinafter be described in the following non-limitative Examples.

Exampl 1:

(1) Polymerization of polyglycolic acid and production of suture:

Two kilograms of glycolide (melting point: 83.5 to 84.5°C) were charged in a thick-wall cylindrical stainless steel polymerization vessel having a separable structure (that is, the main body and cover are separable from each other), followed by the addition of 10 m$\ell$ of a toluene solution of 0.06 g (0.003 wt.%, 1.5 x 10$^{-4}$ mole) of stannous octoate and 5.4 g (0.27 wt.%, 2.9 x 10$^{-2}$ mole, 90 times in weight the stannous octoate) of lauryl alcohol. After evacuation of the vessel for 2 hours in a vacuum of 1 - 5 mmHg (1.33 to 6.66 mbar) the interior of the vessel was filled with nitrogen gas.

The resulting mixture was heated and polymerized for 3 hours at 230 - 235°C in a nitrogen gas atmosphere by means of a mantle heater. After completion of polymerization, the polymerization mixture was drawn from the vessel through a lower part thereof and was led to a pelletizer, where it was pelletized to obtain 1.8 kg of colorless polyglycolic acid.

Its intrinsic viscosity [$\eta$] and melt index MI measured by the methods described above were 1.0 and 2.2 respectively.

The pellets were melt-spun at an extrusion pressure of 100 kg/cm$^2$ (10 N/mm$^2$) and an extrusion temperature of 245°C through a nozzle with 30 holes by means of a conventional melt extruder. The resultant multifilaments were stretched by a factor of 4 on a hot plate held at 120°C. As a result, good multifilaments having a tensile strength of 6.5 g/denier (58.5 g/tex) were obtained.

These multifilaments were then braided to obtain a suture of American standard size 2-0.

The intrinsic viscosity [$\eta$] of the suture was found to be 0.9, while its linear tensile strength was found to be 6.49 kg.

Incidentally, the linear tensile strength was indicated in terms of breaking strength when a 2-cm long specimen of the suture was pulled straight at a crosshead speed of 100 mm/minutes by means of a conventional strength testing machine.

(2) Packing of the suture:

After sterilizing the above-prepared polyglycolic acid suture with ethylene oxide vapour, it was dried at 70°C for 3 hours in vacuum and the surrounding atmosphere was substituted with dry air from a commercial dry air bomb (moisture content: 0.1 wt.%).

The suture sterilized and dried as described above was placed in a commercial catgut package ("Scotch Pack") which had been stored in dry air in advance. While introducing dry air into the package, the suture was hermetically packed.

In order to obtain a comparative sample, a suture was provided from polyglycolic acid, which had been produced in accordance with the procedure of Example 1 of Japanese Patent Publication No. 13595/1970, by conducting its spinning and braiding in the same manner as in the procedure (1) of the present Example. Its intrinsic viscosity [$\eta$] and linear tensile strength were 0.80 and 6.43 kg respectively. The suture was hermetically sealed within a "Scotch Pack" in the same menner as the packing of the suture of this invention.

(3) Solubility test of suture after storage:

The packages thus-obtained with their respective sutures sealed hermetically therein were stored, as they were, for 5 weeks under storage conditions of 55.6°C and 10% relative humidity (one week of such storage is equivalent of 1 year storage at 22°C) and were then opened. The sutures were then dipped in physiological saline of 37°C. One, two, three and four weeks later, the sutures were pulled out of the physiological saline and their linear tensile strengths were measured. Results are given in Table 1.

As apparent from Table 1, it was found that the suture having the intrinsic viscosity [$\eta$] of 0.8 after its 5-week storage had almost no residual tensile strength 4 weeks later and did not function at all as a suture. In contrast, with the suture having the intrinsic viscosity [$\eta$] of 0.9, it was found that an extremely small amount of moisture did not practically affect its function and it was still usable satisfactorily even after passage of a shelf life similar to those of usual commercial products.

Table 1

| | [η] of suture | Storage in weeks | Package | Residual tensile strength (%) | | | | |
|---|---|---|---|---|---|---|---|---|
| | | | | Weeks 0 | 1 | 2 | 3 | 4 |
| Suture of this invention | 0.9 | 0 | - | 100 | 100 | 68 | 26 | 4 |
| Suture of this invention | 0.9 | 5 | "Scotch Pack" | 100 | 98 | 63 | 28 | 3 |
| Suture of this invention | 0.9 | 5 | Aluminum foil-polyethylene laminate | 100 | 98 | 65 | 30 | 4 |
| Suture of comparative sample | 0.8 | 5 | "Scotch Pack" | 100 | 77 | 27 | 10 | 0.1 |

6

EP 0 248 639 B1

Example 2:

(1) Copolymerization of glycolic acid and production of suture:

Charged in a thick-wall cylindrical stainless steel polymerization vessel of the aforesaid separable structure were 1.8 kg of glycolide (melting point: 83.5 - 84.5°C) and 0.2 kg of ℓ-lactide, followed by addition of 10 mℓ of a toluene solution of 0.06 g (0.003 wt.%, $1.5 \times 10^{-4}$ mole) of stannous octoate and 5.4 g (0.27 wt.%, $2.9 \times 10^{-2}$ mole, 90 times in weight the stannous octoate) of lauryl alcohol. After evacuation of the vessel for 2 hours in vacuum of 1 to 5 mmHg (1.33 to 1.66 mbar), the interior of the vessel was filled with nitrogen gas.

The resulting mixture was heated and polymerized for 3 hours at 220 - 230°C in a nitrogen gas atmosphere by means of a mantle heater. After completion of the polymerization, the polymerization mixture was drawn out through a lower part of the vessel and was fed to a pelletizer, where it was pelletized to obtain 1.8 kg of a copolymer of glycolic acid and ℓ-lactic acid. As a result of an NMR measurement of the copolymer, it was found to comprise 91 mole % of glycolic acid moieties and 9 mole % of lactic acid moieties.

Its intrinsic viscosity [$\eta$] and melt index MI measured by the methods described above were 0.95 and 2.5 respectively.

The pellets were melt-spun at an extrusion pressure of 100 kg/cm$^2$ (10 N/mm$^2$) and an extrusion temperature of 230°C through a nozzle with 30 holes by means of a usual melt extruder. The resultant multifilaments were stretched by a factor of 4 on a hot plate at 120°C. As a result, good multifilaments having a tensile strength of 6.3 g/denier (56.7 g/tex) were obtained.

Those multifilaments were then braided to obtain a suture of American standard size 2-0.

The intrinsic viscosity [$\eta$] of the suture was found to be 0.89, while its linear tensile strength was found to be 6.12 kg, measured as indicated hereinbefore.

(2) Packing of the suture:

After sterilizing with ethylene oxide vapor the glycolic acid copolymer suture obtained by the above procedure (1) was dried at 70°C for 3 hours in vacuum and then this atmosphere was changed to dry air from a commercial dry air bomb (moisture content: 0.1%). The suture sterilized and dried as described above was placed in a commercial catgut package ("Scotch Pack") which had been stored in dry air in advance. While introducing dry air into the package, the suture was hermetically packed.

In order to obtain a comparative sample, a suture was provided from a glycolic acid-ℓ-lactic acid copolymer, which had been produced in accordance with the procedure of Example 1 of Japanese Patent Publication No. 14688/1981, by conducting its spinning and braiding in the same manner as in the procedure (1) of the present Example. Its intrinsic viscosity [$\eta$] and linear tensile strength were 0.81 and 6.13 kg respectively. The suture was hermetically sealed within a "Scotch Pack" in the same manner as the packing of the suture of this invention.

(3) Solubility test of suture after storage:

The thus-obtained packages with their respective sutures sealed hermetically therein were stored, as they were, for 5 weeks under storage conditions of 55.6°C and 10% relative humidity and were then opened. The sutures were then dipped in physiological saline of 37°C. One, two, three and four weeks later, the sutures were pulled out of the physiological saline and their linear tensile strengths were measured. Results are given in Table 2.

As apparent from Table 2, it was found that the suture having the intrinsic viscosity [$\eta$] of 0.81 after its 5-week storage had almost no residual tensile strength 4 weeks later and did not function at all as a suture. In contrast, in the case of the suture having the intrinsic viscosity [$\eta$] of 0.9, it was found that an extremely small amount of moisture did not practically affect its function and it was still usable satisfactorily even after passage of a shelf life similar to those of usual commercial products.

Table 2

| Suture | [η] of suture | Storage in weeks | Package | Residual tensile strength (%) | | | | |
|---|---|---|---|---|---|---|---|---|
| | | | Weeks | 0 | 1 | 2 | 3 | 4 |
| Suture of this invention | 0.89 | 0 | — | 100 | 100 | 63 | 21 | 4 |
| Suture of this invention | 0.89 | 5 | "Scotch Pack" | 100 | 98 | 60 | 25 | 4 |
| Suture of this invention | 0.89 | 5 | Aluminum foil-polyethylene laminate | 100 | 98 | 62 | 28 | 3 |
| Suture of comparative sample | 0.81 | 5 | "Scotch Pack" | 100 | 69 | 20 | 3 | 0 |

## Claims

1. A method for storing a suture, the method comprising selection of a suture obtained by spinning a polyglycolic acid polymer or a glycolic acid copolymer of glycolide and ℓ-lactide in an amount not

8

greater than 15% by weight based on the glycolide, said suture having an intrinsic viscosity of at least 0.85; sterilizing and drying the suture; and hermetically packaging the suture together with dry air having a moisture content of 0.1% by weight or below in a container which had been stored in dry air in advance, the container being made of a substantially moisture-impervious material, but not a completely moisture-impervious material such as aluminum foil.

2. The method according to claim 1, wherein the selected suture has been obtained by spinning a polyglycolic acid polymer, the latter having been obtained by polymerizing glycolide at 220 - 240°C in the presence of stannous octoate in an amount of 0.001 - 0.005 wt.% based on the glycolide and lauryl alcohol in an amount 60 - 120 times in weight the stannous octoate.

3. The method according to claim 1 or claim 2, wherein the selected suture has been obtained by spinning of the polyglycolic acid polymer at 235 - 245°C.

4. The method according to claim 2, wherein the intrinsic viscosity of the selected suture spun from the polyglycolic acid polymer is 0.85 - 0.95.

5. The method according to claim 1 or claim 2, wherein the selected suture has been obtained by spinning a glycolic acid copolymer, the latter having been obtained by polymerizing glycolide and ℓ-lactide in the amount not greater than 15% by weight based on the glycolide at 220 - 240°C in the presence of stannous octoate in an amount of 0.001 - 0.005 wt.% based on the glycolide and ℓ-lactide, and lauryl alcohol in an amount 60 - 120 times in weight the stannous octoate.

6. The method according to claim 5, wherein the selected suture has been obtained by spinning of the glycolic acid copolymer at 225 - 235°C.

7. The method according to claim 5, wherein the intrinsic viscosity of the selected suture spun from the glycolic acid copolymer is 0.85 - 0.95.

**Patentansprüche**

1. Verfahren zur Lagerung eines Nahtmaterials, **dadurch gekennzeichnet, daß** man ein Nahtmaterial auswählt, das durch Verspinnen eines Polyglycolsäure-Polymeren oder eines Glycolsäure-Copolymeren aus Glycolid und ℓ-Lactid in einer Menge von nicht mehr als 15 Gew.-%, bezogen auf das Glycolid, erhalten wurde, wobei das Nahtmaterial eine intrinsische Viskosität von mindestens 0,85 besitzt; das Nahtmaterial sterilisiert und trocknet; und das Nahtmaterial zusammen mit trockener Luft mit einem Feuchtigkeitsgehalt von 0,1 Gew.-% oder darunter in einem Behältnis, das zuvor in trockener Luft gelagert wurde, hermetisch verpackt, wobei das Behältnis aus im wesentlichen feuchtigkeitsundurchlässigem, aber nicht vollständig feuchtigkeitsundurchlässigem Material, wie einer Aluminiumfolie, hergestellt ist.

2. Verfahren nach Anspruch 1, **dadurch gekennzeichnet, daß** das ausgewählte Nahtmaterial durch Verspinnen eines Polyglycolsäure-Polymeren erhalten wurde, wobei das letztere durch Polymerisieren von Glycolid bei 220 - 240°C in Gegenwart von Zinn(II)-octoat in einer Menge von 0,001 - 0,005 Gew.-%, bezogen auf das Glycolid, und Laurylalkohol in einer Menge des 60 - 120-fachen des Gewichts des Zinn(II)-octoats erhalten wurde.

3. Verfahren nach Anspruch 1 oder 2, **dadurch gekennzeichnet, daß** das ausgewählte Nahtmaterial durch Verspinnen des Polyglycolsäure-Polymeren bei 235- 245°C erhalten wurde.

4. Verfahren nach Anspruch 2, **dadurch gekennzeichnet, daß** die intrinsische Viskosität des ausgewählten, aus dem Polyglycolsäure-Polymeren gesponnenen Nahtmaterials 0,85 - 0,95 beträgt.

5. Verfahren nach Anspruch 1 oder 2, **dadurch gekennzeichnet, daß** das ausgewählte Nahtmaterial durch Verspinnen eines Glycolsäure-Copolymeren erhalten wurde, wobei das letztere durch Polymerisieren von Glycolid und ℓ-Lactid in einer Menge von nicht mehr als 15 Gew.-%, bezogen auf das Glycolid, bei 220 - 240°C in Gegenwart von Zinn(II)-octoat in einer Menge von 0,001 - 0,005 Gew.-%, bezogen auf das Glycolid und das ℓ-Lactid, und Laurylalkohol in einer Menge des 60 - 120-fachen des

EP 0 248 639 B1

Gewichts des Zinn(II)-octoats erhalten wurde.

6.  Verfahren nach Anspruch 5, **dadurch gekennzeichnet, daß** das ausgewählte Nahtmaterial durch Verspinnen des Glycolsäure-Copolymeren bei 225 - 235°C erhalten wurde.

7.  Verfahren nach Anspruch 5, **dadurch gekennzeichnet, daß** die intrinsische Viskosität des ausgewählten, aus dem Glycolsäure-Copolymeren gesponnenen Nahtmaterials 0,85 - 0,95 beträgt.

**Revendications**

1.  Un procédé pour stocker un fil de suture, la procédé comprenant la choix d'un fil de suture obtenu par filage d'un polymère fait d'acide polyglycolique ou d'un copolymère d'acide glycolique fait de glycolide et de l-lactide an une proportion ne dépassant pas 15 % en poids relativement au glycolide, ledit fil de suture ayant une viscosité intrinsèque d'au moins 0,85 ; la stérilisation et le séchage du fil de suture ; et l'emballage hermétique du fil de suture avec de l'air sec, ayant une teneur en humidité de 0,1 % en poids ou moins, dans un récipient que l'on a conservé préalablement dans de l'air sec, le récipient étant fait d'une matière sensiblement imperméable à l'humidité, mais non d'une matière complètement imperméable à l'humidité telle qu'une pellicule d'aluminium.

2.  Le procédé selon la revendication 1, dans lequel le fil de suture choisi est obtenu par filage d'un polymère fait d'acide polyglycolique, ce dernier étant obtenu par polymérisation de glycolide entre 220 et 240°C, en présence d'octoate stanneux en une proportion de 0,001 à 0,005 % en poids relativement au glycolide, et d'alcool laurylique en une quantité égale à 60 à 120 fois le poids de l'octoate stanneux.

3.  Le procédé selon la revendication 1 ou la revendication 2, dans lequel le fil de suture choisi a été obtenu par filage du polymère fait d'acide polyglycolique entre 235 et 245°C.

4.  Le procédé selon la revendication 2, dans lequel la viscosité intrinsèque du fil de suture choisi filé à partir du polymère fait d'acide polyglycolique est de 0,85 à 0,95.

5.  Le procédé selon la revendication 1 ou la revendication 2, dans lequel le fil de suture choisi a été obtenu par filage d'un copolymère fait d'acide glycolique, ce dernier ayant été obtenu par polymérisation de glycolide et de l-lactide en une proportion ne dépassant pas 15 % en poids relativement au glycolide, entre 220 et 240°C en présence d'octoate stanneaux en une proportion de 0,001 à 0,005 % en poids relativement au glycolide et au l-lactide, et d'alcool laurylique en une quantité de 60 à 120 fois le poids de l'octoate stanneux.

6.  La procédé selon la revendication 5, dans lequel le fil de suture choisi a été obtenu par filage du copolymère d'acide glycolique entre 225 et 235°C.

7.  Le procédé selon la revendication 5, dans lequel la viscosité intrinsèque du fil de suture choisi filé à partir du copolymère d'acide glycolique est de 0,85 à 0,95.